# EUROPEAN PATENT APPLICATION

(11) **EP 4 512 450 A1**
(43) Date of publication of application: **26.02.2025**
(21) Application number: 23791304.1
(22) Date of filing: 20.04.2023
(51) Int. Cl.: A61M 19/00, A61M 5/20, A61M 5/145, A61M 5/31, A61M 5/315

(54) **HYDRAULIC DRIVING TYPE ANESTHESIA BOOSTER**

(30) Priority: 20.04.2022 CN 202210413056
(71) Applicant: CHANGZHOU SIFARY MEDICAL TECHNOLOGY CO., LTD., Changzhou, Jiangsu 213000 (CN)
(72) Inventor: YANG, Mingbo, hangzhou, Jiangsu 213000 (CN); WANG, Hongcan, hangzhou, Jiangsu 213000 (CN); ZHANG, Kai, hangzhou, Jiangsu 213000 (CN); ZHANG, Liyu, hangzhou, Jiangsu 213000 (CN); WANG, Yuan, hangzhou, Jiangsu 213000 (CN)
(74) Representative: Argyma
(86) International application number: PCT/CN2023/089371
(87) International publication number: WO 2023/202650

(57) **Abstract**

The present invention relates to the technical field of anesthesia boosters, and in particular, to a hydraulic driving type anesthesia booster, comprising: a main unit comprising a driving cylinder and a pushing block, the driving cylinder being internally provided with a first cavity and provided with a medium outlet; the pushing block sliding in the first cavity; in the first cavity, a flow medium being filled between the medium outlet and the pushing block; a handle comprising an execution cylinder, a medicine bottle seat and a piston rod, the execution cylinder being internally provided with a second cavity, and one end of the execution cylinder being provided with a medium inlet; the medicine bottle seat being used for placing a medicine bottle, one end of the medicine bottle seat being detachably connected to the execution cylinder, and the other end of the medicine bottle seat being detachably connected to a needle head; the piston rod being slidably arranged in the second cavity, one end of the piston rod extending to the outer side of the execution cylinder and abutting against the medicine bottle, and the other end of the piston rod being provided with an attaching part attached to the inner wall of the second cavity; and a guide pipe used for communicating the medium outlet with the medium inlet. The hydraulic driving type anesthesia booster of the present invention can improve the operation hand feeling of a doctor and reduce the use cost of the device.

## Description

### TECHNICAL FIELD

The present disclosure relates to the field of anesthesia booster technologies, and in particular to a hydraulic driving type anesthesia booster.

### BACKGROUND

During the injection of an oral anesthetic, a patient may suffer from intensive pain if the drug is not injected slowly and evenly. Traditionally, metering and injection pressure are controlled according to a hand-pushing force and doctors' experiences, which, during injection of the anesthetic, may easily result in excessive pressure causing problems such as tissue damages and pain to the patient. Thus, there emerged an anesthesia booster that controls the injection pressure and metering of the anesthetic in a quantitative and constant-pressure manner by pushing a vial by means of a mechanical driving unit to help the doctors solve this problem.

At present, there are mainly two kinds of anesthesia boosters in the market, namely, a hand-held anesthesia booster and a bench anesthesia booster.

The hand-held anesthesia booster having all components mounted on a handle is convenient to carry as a whole. However, since the components such as a vial and a driving unit are all concentrated on the handle, the handle may be overweight, and it is inconvenient to control injection, which may easily affect the stability of the doctors' hands and thus lead to a poor anesthesia effect.

In the bench anesthesia booster, a vial, a driving unit and the like are arranged on a main unit, the main unit may be placed on a bench, and then a needle tip and the vial are connected by a conduit, such that the weight of a hand-held part can be reduced. However, since a liquid medicine passes through the conduit, the conduit can only be used once, and the needle tip, the conduit and the vial need to be replaced every injection. Moreover, due to the long length of the conduit, more liquid medicine remaining in the conduit may cause waste, thereby resulting in a higher use cost of the whole device.

In view of the above problems, based on the rich practical experience and professional knowledge engaged in the engineering application of such products for many years, and with the application of theories, the designer actively studies and innovates to design a hydraulic driving type anesthesia booster, which can improve the operation hand feelings of the doctors and reduce the use cost of the device.

The information disclosed in the Background is intended only to deepen the understanding of the general background of the present disclosure, and should not be taken to acknowledge or in any way imply that the information constitutes the prior art already known to those skilled in the art.

### SUMMARY OF

An objective of the present disclosure is to provide a hydraulic driving type anesthesia booster with respect to the defects in the prior art, for solving the problems that a conventional anesthesia booster is more inconvenient for a doctor to use and the use cost of the device is high.

In order to fulfill the above objective, a technical solution adopted by the present disclosure is that the hydraulic driving type anesthesia booster includes:
a main unit, where the main unit includes a driving cylinder and a pushing block; a first cavity is provided in the driving cylinder and the driving cylinder is provided with a medium outlet communicating outside with the first cavity; the pushing block slides in the first cavity close to or away from the medium outlet; and the first cavity is filled with a flow medium from the medium outlet to the pushing block;
a handle, where the handle includes an actuating cylinder, a vial holder and a piston rod; a second cavity is provided in the actuating cylinder and one end of the actuating cylinder is provided with a medium inlet for communicating the outside with the second cavity; the vial holder is configured to place a vial, one end of the vial holder is detachably connected to an other end of the actuating cylinder, and an other end of the vial holder is detachably connected to a syringe needle; the piston rod is slidably arranged in the second cavity, one end of the piston rod extends to an outer side of the actuating cylinder and abuts against the vial, and an other end of the piston rod is provided with an attaching portion attached to an inner wall of the second cavity; and
a conduit, wherein the conduit is configured to communicate the medium outlet with the medium inlet.

Further, the handle also includes an elastic mechanism, and the elastic mechanism sleeves a part of the piston rod disposed inside the second cavity.

Further, the main unit also includes a pressure gauge arranged at one end of the first cavity for monitoring pressure information of the flow medium.

Further, the main unit also includes a driving circuit board for collecting pressure information monitored by the pressure gauge and controlling a sliding velocity of the pushing block according to the pressure information to keep the pressure information within a set interval.

Further, the main unit also includes a power seat, a threaded sleeve, a power unit and a lead screw; the power seat is detachably connected to one end of the driving cylinder; the threaded sleeve rotates along a fixed axis on the power seat and is driven to rotate by the power unit; the lead screw is screwed into the threaded sleeve, one end of the lead screw is fixedly connected to the pushing block, and the lead screw is provided with a sliding groove extending in a length direction; and the main unit further includes a locating pin fixedly mounted on the power seat, and one end of the locating pin extends into the sliding groove.

Further, the elastic mechanism includes a first spring sleeving the piston rod, and two ends of the first spring respectively abut against an end face of an inner wall of the second cavity and an end face of the attaching portion.

Further, the elastic mechanism includes a connecting sleeve, a second spring and a third spring all sleeving the piston rod; the connecting sleeve is provided with a first abutting edge and a second abutting edge; two ends of the second spring respectively abut against an end face of an inner wall of the second cavity and the first abutting edge; the second abutting edge extends into the second spring; and two ends of the third spring respectively abut against an end face of the attaching portion and the second abutting edge.

Further, at least one of an end face of an inner wall of the second cavity and an end face of the piston rod is provided with a clamping groove for clamping an end portion of the elastic mechanism.

Further, the medium inlet is arranged at an end portion of the actuating cylinder, a center line of the medium inlet coincides with a center line of the piston rod, and the flow medium pushes an end portion of the piston rod when entering from the medium inlet.

Further, an end of the medium inlet close to the piston rod is provided with a gradually expanding section, and the gradually expanding section is of a hole structure gradually expanding toward the piston rod.

Further, a side face of the attaching portion is provided with a first annular groove; a first sealing ring is provided in the first annular groove; and an end face of the attaching portion facing the medium inlet is provided with a plurality of first through holes, and each of the plurality of first through holes is communicated with the first annular groove.

Further, the pushing block includes a rear block body and a front block body; an end face of the front block body facing the rear block body is provided with a clamping portion, and the clamping portion extends into the rear block body; a side face of the rear block body is provided with a second annular groove; a second sealing ring is provided in the second annular groove; a side face of the front block body is provided with a third annular groove; a third sealing ring is provided in the third annular groove ; and an end face of the front block body facing the medium outlet is provided with a plurality of second through holes, and each of the plurality of second through holes is communicated with the second annular groove.

By the technical solution of the present disclosure, the following technical effects can be achieved.

According to the present disclosure, by placing the vial on the handle, and pushing the piston rod in the handle by a fluid medium pushed out of the main unit, a rubber sealing plug on the vial is pushed finally, thereby completing an anesthetic boosting operation. According to the present disclosure, the vial is refillable and replaceable. Compared with a conventional bench anesthetic pusher, the present disclosure only requires replacement of the syringe needle and an anesthetic vial itself, and the conduit can be used continuously, thus greatly reducing the use cost. Compared with a conventional hand-held anesthetic pusher, driving elements of large sizes and weights are all placed on the main unit, and a hydraulic driving solution is adopted such that only one actuating cylinder needs to be placed on the handle, which can reduce the length and the weight of the handle as much as possible and makes it convenient for doctors and nurses to use.

### BRIEF DESCRIPTION OF DRAWINGS

For clearer descriptions of the technical solutions in the embodiments of the present disclosure or in the prior art, the following briefly introduces the accompanying drawings required for describing the embodiments or the prior art. Apparently, the accompanying drawings in the following description show merely some embodiments of the present disclosure, and a person of ordinary skill in the art may still derive other drawings from these accompanying drawings without creative efforts.
FIG. 1 is a schematic structural diagram of a hydraulic driving type anesthesia booster according to an embodiment of the present disclosure;
FIG. 2 is an exploded diagram of a main unit according to an embodiment of the present disclosure;
FIG. 3 is a side sectional view of the main unit according to an embodiment of the present disclosure;
FIG. 4 is an enlarged diagram of A in FIG. 3 according to an embodiment of the present disclosure;
FIG. 5 is an exploded diagram of a handle according to an embodiment of the present disclosure;
FIG. 6 is a side sectional view of a handle in Embodiment 1 according to an embodiment of the present disclosure;
FIG. 7 is an enlarged diagram of B in FIG. 6 according to an embodiment of the present disclosure;
FIG. 8 is a side section view of a handle in Embodiment 2 according to an embodiment of the present disclosure; and
FIG. 9 is an enlarged diagram of C in FIG. 8 according to an embodiment of the present disclosure.

Reference numerals: main unit 1, driving cylinder 11, first cavity 111, medium outlet 112, pushing block 12, rear block body 121, front block body 122, clamping portion 123, second annular groove 124, second sealing ring 125, third annular groove 126, third sealing ring 127, second through hole 128, pressure gauge 13, driving circuit board 14, power seat 15, threaded sleeve 16, power unit 17, lead screw 18, siding groove 181, locating pin 19, handle 2, actuating cylinder 21, second cavity 211, medium inlet 212, gradually expanding section 213, vial holder 22, piston rod 23, attaching portion 231, first annular groove 232, first sealing ring 233, first through hole 234, elastic mechanism 24, first spring 241, connecting sleeve 242, second spring 243, third spring 244, first abutting edge 245, second abutting edge 246, clamping groove 25, conduit 3.

### DETAILED DESCRIPTION

The technical solutions of the embodiments of the present disclosure will be described clearly and completely with reference to the accompanying drawings in the embodiments of the present disclosure. Apparently, the described embodiments are only part of embodiments of the present disclosure, rather than all of the embodiments.

In the descriptions of the present disclosure, it should be noted that orientation or positional relationships indicated by the terms "center", "upper", "lower", "left", "right", "vertical", "horizontal", "inside", "outside", etc. are orientation or positional relationships shown on the basis of the drawings, only for the purposes of the ease in describing the present disclosure and simplification of its descriptions, but not indicating or implying that the specified device or element has to be specifically located, and structured and operated in a specific direction, and therefore, should not be understood as limitations to the present disclosure.

In the descriptions of the present disclosure, it should be noted that unless otherwise explicitly defined or limited, the terms "mounted", "connected with" and "connected to" should be comprehended in a broad sense. For example, they may refer to a fixed connection, detachable connection or integrated connection, or may be a mechanical connection or electrical connection, or may refer to a direct connection or an indirect connection via an intermediary, or may be an internal communication of two elements. The specific meanings about the foregoing terms in the present disclosure can be understood by those skilled in the art according to specific circumstances.

### Embodiment 1

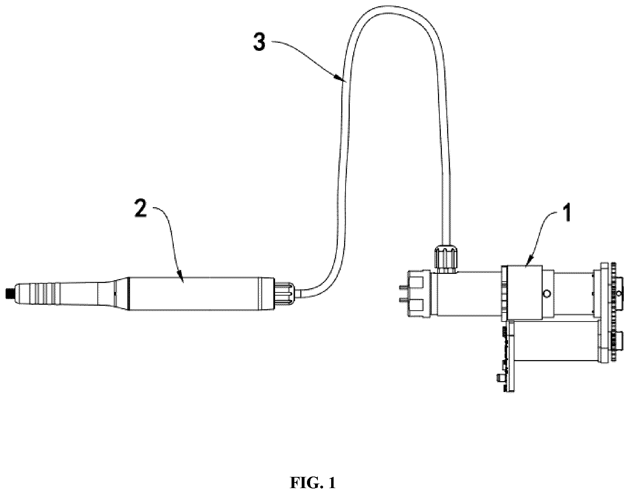

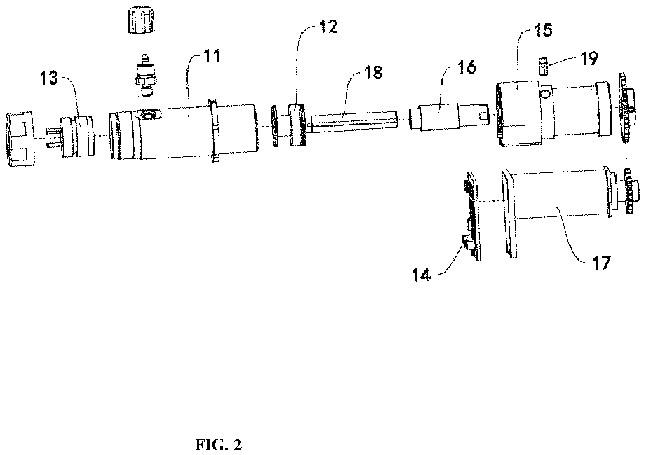

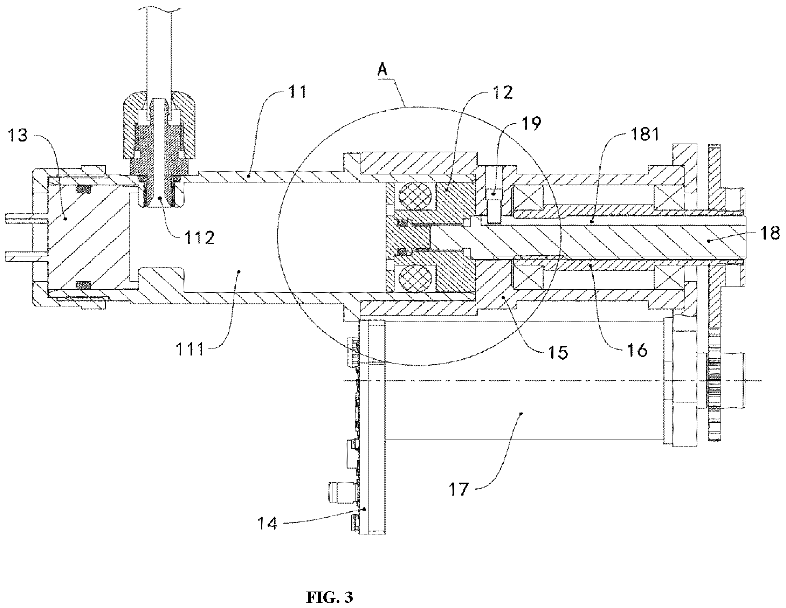

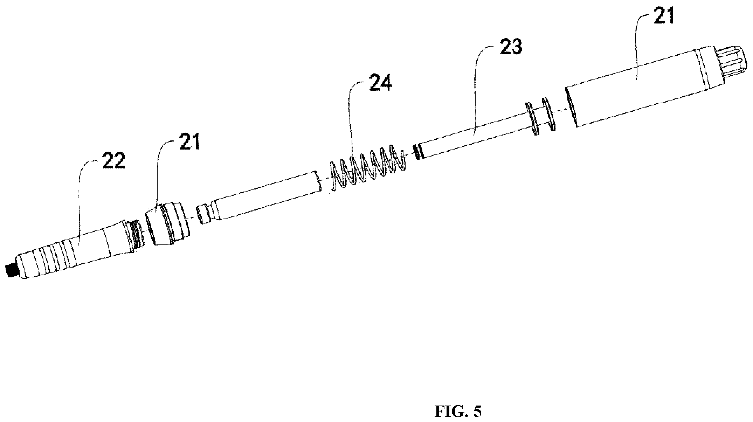

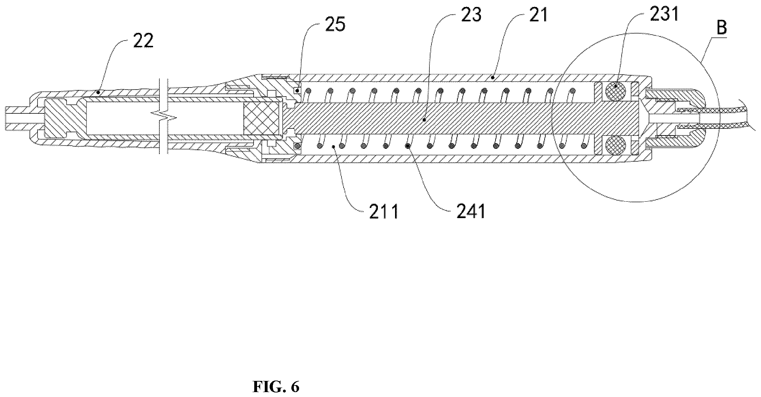

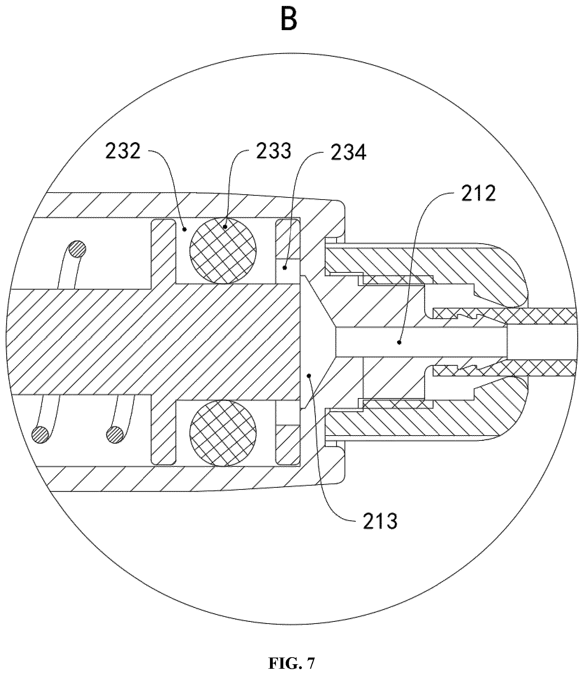

As shown in FIGS. 1-7, a hydraulic driving type anesthesia booster includes a main unit 1, a handle 2 and a conduit 3.

The main unit 1 includes a driving cylinder 11 and a pushing block 12. A first cavity 111 is provided in the driving cylinder 11 and the driving cylinder 11 is provided with a medium outlet 112 communicating the outside with the first cavity 111. The pushing block 12 slides in the first cavity 111 close to or away from the medium outlet 112. The first cavity 111 is filled with a flow medium from the medium outlet 112 to the pushing block 12, and the flow medium is a liquid such as pressure oil, water and alcohol, and is most preferably pressure oil for optimal force transfer.

The handle 2 includes an actuating cylinder 21, a vial holder 22 and a piston rod 23. A second cavity 211 is provided in the actuating cylinder 21 and one end of the actuating cylinder 21 is provided with a medium inlet 212 for communicating the outside with the second cavity 211. The vial holder 22 is configured to place a vial, one end of the vial holder 22 is detachably connected to the other end of the actuating cylinder 21, and the other end of the vial holder 22 is provided with a needle inserting port and is detachably connected to a syringe needle. After the syringe needle is mounted, a tail portion of the syringe needle may extend into the vial holder 22 and insert into the vial, such that a liquid medicine in the vial may flow into the syringe needle. The detachable installation mode of the two places may be implemented through various forms such as screw fixation, thread-sleeve matching and tightening and the like. The piston rod 23 is slidably arranged in the second cavity 211, one end of the piston rod 23 extends to an outer side of the actuating cylinder 21 and abuts against a rubber sealing plug of the vial, and the other end of the piston rod 23 is provided with an attaching portion 231 attached to an inner wall of the second cavity 211.

The conduit 3 is configured to communicate the medium outlet 112 with the medium inlet 212.

Preferably, the handle 2 is also provided with an elastic mechanism 24, and the elastic mechanism 24 sleeves a part of the piston rod 23 disposed inside the second cavity 211 for assisting the piston rod 23 in quick resetting.

Specifically, the working principle of the anesthesia booster is described as below. In the main unit 1, by sliding the pushing block 12 toward the medium outlet 112, the pushing block 12 may push the flow medium out of the medium outlet 112, the flow medium flows into the medium inlet 212 along the conduit 3, and then the attaching portion 231 of the piston rod 23 is pushed to cause the piston rod 23 to move toward the vial as a whole, such that the rubber sealing plug on the vial can be pushed, the elastic mechanism 24 can be compressed, and finally the liquid medicine in the vial can be pushed out from the syringe needle. Upon completion of the injection of the liquid medicine, the pushing block 12 slides away from the medium outlet 112 to suck the flow medium back into the driving cylinder 11 through the conduit 3. Besides, the compressed elastic mechanism 24 may also generate an elastic deformation force to push the piston rod 23 to reset, and the flow medium is pushed out of the actuating cylinder 21 to assist the flow medium in returning to the driving cylinder 11. After that, the vial holder 22 is removed, and the vial and the syringe needle are replaced for the next round of anesthesia. Compared with a conventional bench anesthetic pusher, the present disclosure only requires replacement of the syringe needle and the vial, and the conduit 3 can be used continuously, thus greatly reducing the use cost. Compared with a conventional hand-held anesthetic pusher, driving elements of large sizes and weights are all placed on the main unit 1, and a hydraulic driving solution is adopted such that only one actuating cylinder 21 needs to be placed on the handle 2, which can reduce the length and the weight of the handle 2 as much as possible and makes it convenient for doctors and nurses to use.

In the process of anesthesia, if the pressure of anesthetic injectionis too high, it will lead to tissue damage, patient pain and other problems , and thus it is necessary to monitor the pressure of the flow medium. Preferably, the main unit 1 further includes a pressure gauge 13 arranged at one end of the first cavity 111 for monitoring pressure information of the flow medium, and the pressure information is transmitted to the outside and fed back to the doctor in the form of sound, image, tactile vibration and the like. Alternatively, the main unit 1 may also be provided with a driving circuit board 14, a wiring terminal of the pressure gauge 13 is connected to the driving circuit board 14, and the driving circuit board 14 has a built-in automatic control program for collecting the pressure information monitored by the pressure gauge 13 and accurately controlling the sliding velocity of the pushing block 12 in real time according to the pressure information, so as to keep the pressure information within a set interval and realize less pressure fluctuation, such that a better injection effect can be achieved and the pain of the patient can be relieved.

As a preferred structure for driving the pushing block 12 to move, the main unit 1 further includes a power seat 15, a threaded sleeve 16, a power unit 17 and a lead screw 18. The power seat 15 is detachably connected to one end of the driving cylinder 11. The threaded sleeve 16 rotates along a fixed axis on the power seat 15 and is driven to rotate by the power unit 17. The lead screw 18 is screwed into the threaded sleeve 16, and one end of the lead screw 18 is fixedly connected to the pushing block 12. In this structure, the lead screw 18 can be driven to move forward or backward by a clockwise rotation or counterclockwise rotation of the threaded sleeve 16, so as to push the pushing block 12 to move.

A traditional hydraulic system, which usually realizes movement of the flow medium by means of an oil tank section and a control valve section, is complicated in structure, poor in reliability and excessively low in control accuracy, and hence cannot be adapted to the high-precision use scenarios of medical applications. However, in this structure, the threaded sleeve 16 and the lead screw 18 are in threaded fit for pushing, which achieves high control precision. The threaded fit is self-locking, i.e., the active rotation of the threaded sleeve 16 can drive the lead screw 18 to move, but it is difficult or even impossible for the active movement of the lead screw 18 to drive the threaded sleeve 16 to rotate, such that a precise stop can be realized in the working process, which in turn ensures the accuracy of the amount of anesthetic injection. The power unit 17 can be connected by the driving circuit board 14 to form closed-loop control, such that the pressure of the whole hydraulic system can be automatically and accurately controlled. Moreover, compared with the structure of the traditional hydraulic system, this structure is more concise, and thus it can reduce the maintenance cost and the maintenance difficulty, and makes the maintenance cost lower and the maintenance more convenient. Preferably, the power unit 17 and the threaded sleeve 16 are in transmission connection by means of a deceleration structure, such as a reduction box and a reduction gear set, such that the power unit 17 can control a rotation number of the threaded sleeve 16 more accurately.

Preferably, the lead screw 18 is provided with a sliding groove 181 extending in a length direction. The main unit 1 further includes a locating pin 19 fixedly mounted on the power seat 15, and one end of the locating pin 19 extends into the sliding groove 181. During the rotation of the threaded sleeve 16, the locating pin 19 can effectively prevent the lead screw 18 from rotating along with the threaded sleeve 16, and can make the lead screw 18 move linearly toward the first cavity 111, thereby further ensuring precise control of the threaded sleeve 16 by the power unit 17.

In this embodiment, the elastic mechanism 24 includes a first spring 241 sleeving the piston rod 23, and two ends of the first spring 241 respectively abut against an end face of an inner wall of the second cavity 211 and an end face of the attaching portion 231 facing the vial, such that the function of the elastic mechanism 24 can be achieved by means of the simplest structure, and the manufacturing and maintenance costs can be reduced.

In the handle 2, at least one of the end face of the inner wall of the second cavity 211 and an end face of the piston rod 23 is preferably provided with a clamping groove 25 for clamping an end portion of the elastic mechanism 24, so as to prevent the elastic mechanism 24 from being skewed and affecting normal operation of the handle 2.

Preferably, the medium inlet 212 is arranged at an end portion of the actuating cylinder 21, and a center line of the medium inlet 212 coincides with a center line of the piston rod 23, such that the flow medium, when entering from the medium inlet 212, can push the end portion of the piston rod 23. Besides, the point of action of a main pushing force is disposed on the center line of the piston rod 23, thereby ensuring the stability of movement of the piston rod 23. Preferably, an end of medium inlet 212 close to the piston rod 23 is provided with a gradually expanding section 213. The gradually expanding section 213 is of a hole structure that gradually expands toward the piston rod 23. The gradually expanding section 213 can uniformly diffuse the flow medium after the flow medium passes through the medium inlet 212, such that when the handle 2 is started, the flow medium has a larger area of action on the piston rod 23, thereby preventing the piston rod 23 from shaking due to a sudden concentrated stress, which may affect the hand stability of doctors and nurses. Preferably, a side face of the attaching portion 231 is provided with a first annular groove 232, a first sealing ring 233 is provided in the first annular groove 232 , and the first sealing ring 233 is attached to an inner wall of the actuating cylinder 21 and an inner wall of the first annular groove 232; and an end face of attaching portion 231 facing the medium inlet 212 is provided with a plurality of first through holes 234, and the first through holes 234 are communicated with the first annular groove 232. In this structure, the flow medium can enter the first annular groove 232 through the first through holes 234 to lubricate the first sealing ring 233. Meanwhile, the flow medium with a certain pressure can generate pushing forces on both of the surface of the first sealing ring 233 and the end face of the attaching portion 231, such that the stressed area is larger, and the smooth and steady movement of the piston rod 23 can be further ensured. Preferably, the first through hole 234 is also communicated with the gradually expanding section 213. In the case that the end face of the attaching portion 231 is attached to the end face of the inner wall of the actuating cylinder 21, the flow medium can also enter the first annular groove 232 through the first through hole 234 to push the first sealing ring 233, such that the piston rod 23 can be started quickly.

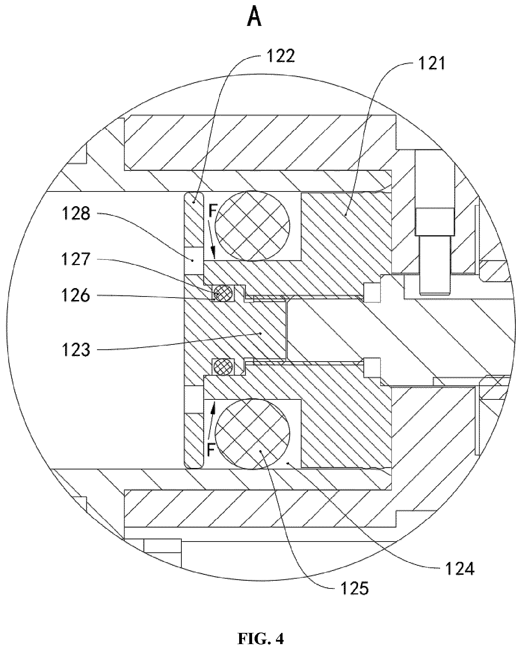

In order to facilitate the manufacture and installation of the pushing block 12, a preferred structure of the pushing block 12 includes a rear block body 121 and a front block body 122, the rear block body 121 is connected to the lead screw 18, an end face of the front block body 122 facing the rear block body 121 is provided with a clamping portion 123, the clamping portion 123 extends into the rear block body 121, and a top end of the clamping portion 123 is provided with threads screwed into the rear block body 121, thus realizing fixation between the rear block body 121 and the front block body 122. Meanwhile, the clamping portion 123 may also increase the area of contact between the rear block body 121 and the front block body 122, so as to keep the relative position between the rear block body 121 and the front block body 122 better fixed, reduce shaking that may be generated during the movement of the rear block body 121 and the front block body 122, and ensure normal operation of the device. Preferably, a side face of the rear block body 121 is provided with a second annular groove 124, a second sealing ring 125 is provided in the second annular groove 124, a side face of the front block body 122 is provided with a third annular groove 126, a third sealing ring 127 is provided in the third annular ring 126, an end face of the front block body 122 facing the medium outlet 112 is provided with a plurality of second through holes 128, the plurality of second through holes 128 are communicated with the second annular groove 124, and the second through holes 128 may also cause the flow medium to enter the second annular groove 124 so as to lubricate the second sealing ring 125. Meanwhile, the flow medium with movement pressure, after entering the second annular groove 124, may generate a pressure F on an inner wall of the second annular groove 124 as shown in FIG. 4. The pressure F may push a wall surface of the rear block body 121 toward the clamping portion 123, such that the rear block body 121 and the clamping portion 123 can be directly attached more tightly. In addition, the pressure F may further compress the third sealing ring 127, such that the third sealing ring 127 can have a better sealing effect.

### Embodiment 2

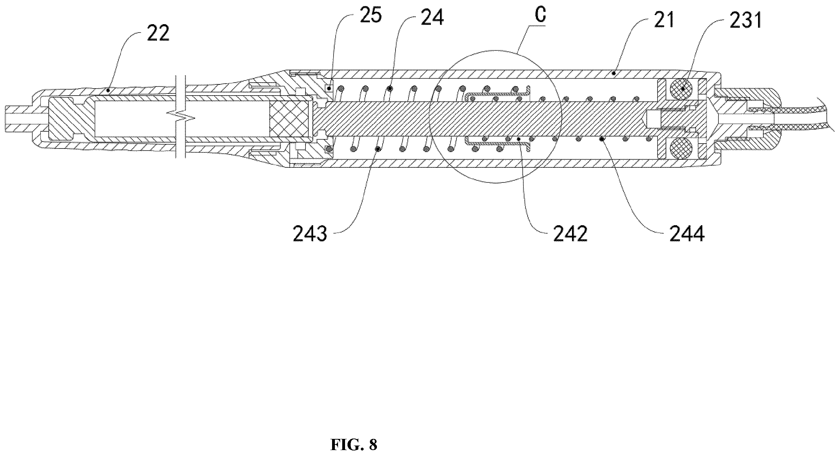

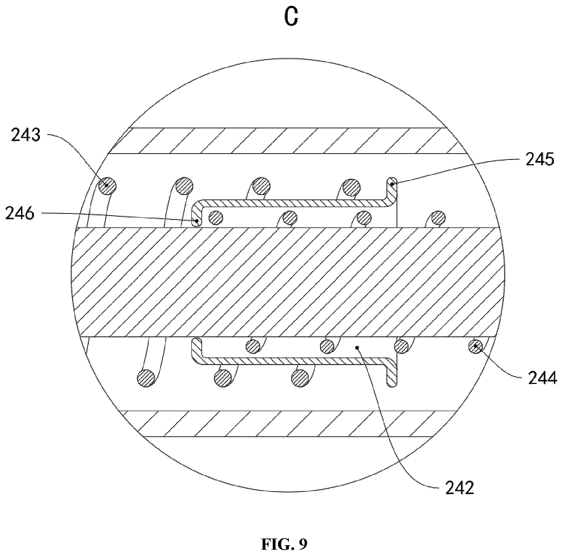

As shown in FIGS. 1-5 and 8-9, a hydraulic driving type anesthesia booster differs from Embodiment 1 in that the elastic mechanism 24 in this embodiment includes a connecting sleeve 242, a second spring 243 and a third spring 244 all sleeving the piston rod 23. The connecting sleeve 242 is provided with a first abutting edge 245 and a second abutting edge 246, two ends of the second spring 243 abut against an end face of an inner wall of the second cavity 211 and the first abutting edge 245 respectively. The second abutting edge 246 extends into the second spring 243, and two ends of the third spring 244 abut against an end face of the attaching portion 231 facing the vial and the second abutting edge 246 respectively. The design principle of this structure is that a part of the third spring 244 may extend into the second spring 243 by means of the connecting sleeve 242, such that the length of the elastic mechanism 24 can be effectively reduced, which in turn can reduce the length of the handle 2 and improve the use comfort of doctors and nurses. Meanwhile, as the spring is compressed more and more, a reaction force of its elastic deformation may be greater and may offset the pushing force of the flow medium when acting on the piston rod 23. With the movement of the piston rod 23, the pushing ability of the flow medium to the piston rod 23 can be greatly reduced. However, in the case where the same compression amount is generated as the elastic mechanism 24 in Embodiment 1, the second spring 243 and the third spring 244 in this embodiment may share the amount of deformation by means of the connecting sleeve 242 in this structure, and the degrees of deformations of the second spring 243 and the third spring 244 are only half of that of the first spring 241, thereby ensuring that deformation forces generated by the second spring 243 and the third spring 244 have less impact on the sliding of the piston rod 23 and further ensuring normal operation of the device.

The basic principles, main features and advantages of the present disclosure have been shown and described as above. It should be understood by those skilled in the art that the present disclosure is not limited by the above-mentioned embodiments, the above embodiments and the description merely illustrate the principles of the present disclosure, there may be various changes and improvements to the present disclosure without departing from the spirit and the scope of the present disclosure, and all these changes and improvements shall fall within the scope of the claimed scope of the present disclosure. The scope of the present disclosure is defined by the appended claim and their equivalents.

## Claims

1. A hydraulic driving type anesthesia booster, comprising:
a main unit (1), wherein the main unit (1) comprises a driving cylinder (11) and a pushing block (12); a first cavity (111) is provided in the driving cylinder (11) and the driving cylinder (11) is provided with a medium outlet (112) communicating outside with the first cavity (111); the pushing block (12) slides in the first cavity (111) close to or away from the medium outlet (112); and the first cavity (111) is filled with a flow medium from the medium outlet (112) to the pushing block (12);
a handle (2), wherein the handle (2) comprises an actuating cylinder (21), a vial holder (22) and a piston rod (23); a second cavity (211) is provided in the actuating cylinder (21) and one end of the actuating cylinder (21) is provided with a medium inlet (212) for communicating the outside with the second cavity (211); the vial holder (22) is configured to place a vial, one end of the vial holder (22) is detachably connected to an other end of the actuating cylinder (21) and an other end of the vial holder (22) is detachably connected to a syringe needle; the piston rod (23) is slidably arranged in the second cavity (211), one end of the piston rod (23) extends to an outer side of the actuating cylinder (21) and abuts against the vial, and an other end of the piston rod (23) is provided with an attaching portion (231) attached to an inner wall of the second cavity (211); and
a conduit (3), wherein the conduit (3) is configured to communicate the medium outlet (112) with the medium inlet (212).

2. The hydraulic driving type anesthesia booster according to claim 1, wherein the handle (2) further comprises an elastic mechanism (24), and the elastic mechanism (24) sleeves a part of the piston rod (23) disposed inside the second cavity (211).

3. The hydraulic driving type anesthesia booster according to claim 1, wherein the main unit (1) further comprises a pressure gauge (13) arranged at one end of the first cavity (111) for monitoring pressure information of the flow medium.

4. The hydraulic driving type anesthesia booster according to claim 3, wherein the main unit (1) further comprises a driving circuit board (14) for collecting pressure information monitored by the pressure gauge (13) and controlling a sliding velocity of the pushing block (12) according to the pressure information to keep the pressure information within a set interval.

5. The hydraulic driving type anesthesia booster according to claim 1, wherein the main unit (1) further comprises a power seat (15), a threaded sleeve (16), a power unit (17) and a lead screw (18); the power seat (15) is detachably connected to one end of the driving cylinder (11); the threaded sleeve (16) rotates along a fixed axis on the power seat (15) and is driven to rotate by the power unit (17); the lead screw (18) is screwed into the threaded sleeve (16), one end of the lead screw (18) is fixedly connected to the pushing block (12), and the lead screw (18) is provided with a sliding groove (181) extending in a length direction; and the main unit (1) further comprises a locating pin (19) fixedly mounted on the power seat (15), and one end of the locating pin (19) extends into the sliding groove (181).

6. The hydraulic driving type anesthesia booster according to claim 2, wherein the elastic mechanism (24) comprises a first spring (241) sleeving the piston rod (23), and two ends of the first spring (241) respectively abut against an end face of an inner wall of the second cavity (211) and an end face of the attaching portion (231).

7. The hydraulic driving type anesthesia booster according to claim 2, wherein the elastic mechanism (24) comprises a connecting sleeve (242), a second spring (243) and a third spring (244) all sleeving the piston rod (23); the connecting sleeve (242) is provided with a first abutting edge (245) and a second abutting edge (246); two ends of the second spring (243) respectively abut against an end face of an inner wall of the second cavity (211) and the first abutting edge (245); the second abutting edge (246) extends into the second spring (243); and two ends of the third spring (244) respectively abut against an end face of the attaching portion (231) and the second abutting edge (246).

8. The hydraulic driving type anesthesia booster according to claim 2, wherein at least one of an end face of an inner wall of the second cavity (211) and an end face of the piston rod (23) is provided with a clamping groove (25) for clamping an end portion of the elastic mechanism (24).

9. The hydraulic driving type anesthesia booster according to claim 1, wherein the medium inlet (212) is arranged at an end portion of the actuating cylinder (21), a center line of the medium inlet (212) coincides with a center line of the piston rod (23), and the flow medium pushes an end portion of the piston rod (23) when entering from the medium inlet (212).

10. The hydraulic driving type anesthesia booster according to claim 9, wherein an end of the medium inlet (212) close to the piston rod (23) is provided with a gradually expanding section (213), and the gradually expanding section (213) is of a hole structure gradually expanding toward the piston rod (23).

11. The hydraulic driving type anesthesia booster according to claim 10, wherein a side face of the attaching portion (231) is provided with a first annular groove (232); a first sealing ring (233) is provided in the first annular groove (232); and an end face of the attaching portion (231) facing the medium inlet (212) is provided with a plurality of first through holes (234), and each of the plurality of first through holes (234) is communicated with the first annular groove (232).

12. The hydraulic driving type anesthesia booster according to claim 1, wherein the pushing block (12) comprises a rear block body (121) and a front block body (122); an end face of the front block body (122) facing the rear block body (121) is provided with a clamping portion (123), and the clamping portion (123) extends into the rear block body (121); a side face of the rear block body (121) is provided with a second annular groove (124); a second sealing ring (125) is provided in the second annular groove (124); a side face of the front block body (122) is provided with a third annular groove (126); a third sealing ring (127) is provided in the third annular groove (126); and an end face of the front block body (122) facing the medium outlet (112) is provided with a plurality of second through holes (128), and each of the plurality of second through holes (128) is communicated with the second annular groove (124).
